# EUROPÄISCHE PATENTSCHRIFT

(11) **EP 3 477 299 B1**
(45) Veröffentlichungstag und Bekanntmachung des Hinweises auf die Patenterteilung: **11.12.2024**
(21) Anmeldenummer: 18207435.1
(22) Anmeldetag: 10.07.2015
(51) Int. Cl.: G01N 33/53, C40B 30/04, B01L 7/00, B01L 3/00, C12Q 1/6811

(54) **VERFAHREN ZUR IDENTIFIZIERUNG HOCHAFFINER KOMPLEXE AUS ZWEI LIGANDEN UND EINEM REZEPTOR, VORRICHTUNG ZUR DURCHFÜHRUNG DES VERFAHRENS UND SELBST-ASSEMBLIERENDE CHEMISCHE BIBLIOTHEK ZUR VERWENDUNG IN DEM VERFAHREN**
METHOD FOR IDENTIFICATION OF HIGH AFFINITY COMPLEXES COMPRISING TWO LIGANDS AND A RECEPTOR, DEVICE FOR CARRYING OUT SAID METHOD AND SELF-ASSEMBLING CHEMICAL LIBRARY FOR USE IN THE METHOD
PROCÉDÉ D'IDENTIFICATION DES COMPLEXES À AFFINITÉ ÉLEVÉE COMPOSÉS DE DEUX LIGANDS ET D'UN RÉCEPTEUR, DISPOSITIF DE MISE EN UVRE DU PROCÉDÉ ET BIBLIOTHÈQUE CHIMIQUE À AUTO-ASSEMBLAGE DESTINÉ À ÊTRE UTILISÉE LORS DU PROCÉDÉ

(30) Priorität: 16.07.2014 DE 102014213783
(43) Veröffentlichungstag der Anmeldung: 01.05.2019
(62) Teilanmeldung aus: 15744501.6
(73) Patentinhaber: DyNAbind GmbH, 01307 Dresden (DE)
(72) Erfinder: REDDAVIDE, Francesco, 01099 Dresden (DE); DE ANDRADE, Helena, 01309 Dresden (DE); LIN, Weilin, 01309 Dresden (DE); ZHANG, Yixin, 01309 Dresden (DE); MELE, Elisa, 73020 Castrignano dei Greci (Lecce) (IT)
(74) Vertreter: Pfenning, Meinig & Partner mbB

(56) Entgegenhaltungen:
- WO-A1-03/076943
- WO-A1-97/43232
- WO-A2-2010/150103
- US-A1- 2010 159 455
- DAGUER JP ET AL: "DNA-templated combinatorial assembly of small molecule fragments amenable to selection/amplification cycles", CHEMICAL SCIENCE, vol. 2, no. 4, 24 January 2011 (2011-01-24), pages 625, XP055212512, ISSN: 2041-6520, DOI: 10.1039/c0sc00574f
- DAGUER JP ET AL: "DNA-templated combinatorial assembly of small molecule fragments amenable to selection/amplification cycles. Supplementary material.", CHEMICAL SCIENCE, vol. 2, no. 4, 1 January 2011 (2011-01-01), pages 625, XP055212494, ISSN: 2041-6520, DOI: 10.1039/c0sc00574f
- CIOBANU M ET AL: "Selection of a synthetic glycan oligomer from a library of DNA-templated fragments against DC-SIGN and inhibition of HIV gp120 binding to dendritic cells", CHEMICAL COMMUNICATIONS, vol. 47, no. 33, 25 July 2011 (2011-07-25), pages 9321, XP055212502, ISSN: 1359-7345, DOI: 10.1039/c1cc13213j
- FRANZINI RM ET AL: "DNA-Encoded Chemical Libraries: Advancing beyond Conventional Small-Molecule Libraries", ACCOUNTS OF CHEMICAL RESEARCH, vol. 47, no. 4, 28 March 2014 (2014-03-28), pages 1247 - 1255, XP055211954, ISSN: 0001-4842, DOI: 10.1021/ar400284t
- SCHEUERMANN J ET AL: "Dual-pharmacophore DNA-encoded chemical libraries", CURRENT OPINION IN CHEMICAL BIOLOGY, vol. 26, 13 March 2015 (2015-03-13), pages 99 - 103, XP055211951, ISSN: 1367-5931, DOI: 10.1016/j.cbpa.2015.02.021
- SCHEUERMANN J ET AL: "DNA-encoded chemical libraries for the discovery of MMP-3 inhibitors", BIOCONJUGATE CHEMISTRY, ACS, WASHINGTON, DC, US, vol. 19, no. 3, 1 March 2008 (2008-03-01), pages 778 - 785, XP002737640, ISSN: 1043-1802, [retrieved on 20080207], DOI: 10.1021/BC7004347
- MANNOCCI L ET AL: "20 years of DNA-encoded chemical libraries", CHEMICAL COMMUNICATIONS > CHEMICAL COMMUNICATIONS - CHEMCOM; [6015D], ROYAL SOCIETY OF CHEMISTRY, GB, vol. 47, no. 48, 28 December 2011 (2011-12-28), pages 12747 - 12753, XP002695069, ISSN: 1359-7345, [retrieved on 20111114], DOI: 10.1039/C1CC15634A
- BULLER F ET AL: "Drug discovery with DNA-encoded chemical libraries", BIOCONJUGATE CHEMISTRY, ACS, WASHINGTON, DC, US, vol. 21, no. 9, 15 September 2010 (2010-09-15), pages 1571 - 1580, XP002695068, ISSN: 1043-1802, [retrieved on 20100803], DOI: 10.1021/BC1001483

## Beschreibung

Es wird ein Verfahren zur Identifizierung von hochaffinen Komplexen aus zwei Liganden und einem Rezeptor vorgeschlagen. In dem Verfahren werden Liganden einer Bibliothek verwendet, die jeweils eine einzelsträngige DNA oder RNA aufweisen. Da die DNA oder RNA von einem Teil der Liganden zu der DNA oder RNA eines anderen Teils der Liganden komplementär ist, können beide Teile der Liganden untereinander binäre Komplexe bilden. Die Länge der DNA oder RNA kann in einer erfindungsgemäßen Ausführungsform 3 bis 10 Basen betragen. Erfindungsgemäß ist eine Hybridisierung und Dissoziation der einzelsträngigen DNAs oder RNAs in dynamischer Art und Weise vorgesehen, das für die erfindungsgemäße Ausführungsform bereits bei Raumtemperatur erreicht wird und für eine nicht-erfindungsgemäße Ausführungsform einen Kreislauf aus Aufheizen und Abkühlen benötigt. Darüber hinaus wird eine mikrofluidische Vorrichtung zur Durchführung des erfindungsgemäßen Verfahrens und eine dynamische, selbst-assemblierende chemische Bibliothek enthaltend binäre Komplexe der jeweiligen Liganden vorgeschlagen.

Eine wesentliche Herausforderung bei der Verwendung von DNA-kodierten chemischen Bibliotheken und DNA/RNA-Aptamer Bibliotheken für das Auffinden von wirksamen Bindern und Inhibitoren gegen verschiedenartige Zielproteine von biomedizinischem Interesse ist die Verbesserung des Signal-Stör-Verhältnisses in Selektionsexperimenten, um Moleküle mit einer hohen Bindungsaffinität von schwachen oder nicht bindenden Verbindungen zu unterscheiden.

Bisher wurden im Stand der Technik meist parallele Selektionsexperimente mit unterschiedlicher Selektionsstringenz realisiert, um die Selektionsbedingungen gegenüber einem bekannten Zielprotein zu optimieren. So wurden zum Beispiel feste Trägermaterialien mit unterschiedlichen Mengen des Zielproteins beladen. Desweiteren wurden verschiedene Waschverfahren etabliert, bei denen Puffer mit unterschiedlichen Ionenstärken und Detergenzien verwendet wurden.

Um parallele Selektionsexperimente durchzuführen, müssen viele zusätzliche Experimente für ein Zielprotein durchgeführt werden. Dies ist sowohl zeitaufwendig als auch kostspielig. Zudem ist es sehr schwierig, parallele Selektionsexperimente mit unterschiedlicher Stringenz in der Selektion zu designen, wenn die Verteilungen der Bindungsaffinitäten der gesamten Bibliothek für das Zielprotein unbekannt sind. Um Binder mit einer hohen Affinität zu selektieren müssen spezielle Selektionsbedingungen angewendet werden. Demnach ist es ein Nachteil, dass ein stringentes, paralleles Selektionsexperiment erst konzipiert werden kann, nachdem bekannt ist, dass sich hochaffine Bindungsmoleküle in der Bibliothek befinden.

Andererseits ist auch die Charakterisierung von Komponenten eines erstmaligen Selektionsexperiments (de-novo-Selektionsexperiment) sehr zeitaufwendig und teuer. Es ist zudem schwierig, ohne das Wissen und die aus einer vorangegangenen primären Selektion resultierenden Informationen ein paralleles Selektionsexperiment zu konzipieren.

Die US 2010/159455 A1 offenbart ein Verfahren zum biologischen Nachweis von Rezeptoren und Proteinen und der Erstellung von Profilen von Rezeptoren und Proteinen.

Die WO 97/43232 A1 offenbart eine Substanzbibliothek, ein Verfahren zur Herstellung der Substanzbibliothek, ein Verfahren zur Herstellung von supramolekularen Komplexen unter Verwendung der Substanzbibliothek, eine Verwendung der hergestellten supramolekularen Komplexe und eine Verwendung der Substanzbibliothek.

Die Aufgabe der vorliegenden Erfindung ist somit die Bereitstellung eines Verfahrens, mit dem auf einfache, ökonomische und hochsensitive Art und Weise eine Identifizierung hochaffiner ternärer Komplexe aus zwei Liganden und einem Rezeptor möglich ist. Zudem sollte eine Vorrichtung zur Durchführung des Verfahrens und eine selbst-assemblierende chemische Bibliothek zur Verwendung in dem Verfahren bereitgestellt werden.

Die Aufgabe wird gelöst durch das Verfahren gemäß Anspruch 1, die dynamische, selbst-assemblierende chemische Bibliothek gemäß Anspruch 10 und die Verwendung der Bibliothek gemäß Anspruch 15.

Es wird ein nicht-erfindungsgemäßes Verfahren zur sensitiven Identifizierung hochaffiner Komplexe aus zwei Liganden und einem Rezeptor bereitgestellt, das folgende Schritte umfasst:
a) Interagieren von einer Vielzahl von verschiedenen Komplexen aus Liganden einer chemischen Bibliothek mit zumindest einem Rezeptor in einer Lösung, wobei die Liganden der Bibliothek eine einzelsträngige DNA oder RNA mit einer Basenlänge von mehr als 10 Basen aufweisen, die chemisch kovalent an die Liganden gebunden ist und wobei mehr als 10 Basen der einzelsträngigen DNA oder RNA von einem ersten Teil der Liganden zu Basen der einzelsträngigen DNA oder RNA von einem zweiten Teil der Liganden komplementär sind und wobei die Liganden über Hybridisierung der DNA oder RNA zu Ligandenkomplexen komplexiert sind;
b) Inkubieren der Lösung für einen bestimmten Zeitraum, wobei Komplexe aus Ligandenkomplexen und dem Rezeptor entstehen;
c) Dissoziieren der Ligandenkomplexe zu freien Liganden;
d) Re-Hybridisierung der freien Liganden zu weiteren Ligandenkomplexen;
e) Inkubieren der Lösung für einen bestimmten Zeitraum, wobei weitere Komplexe aus den weiteren Ligandenkomplexen und dem Rezeptor entstehen;
f) Identifizieren der entstandenen Komplexe aus Ligandenkomplexen und dem Rezeptor.

Das nicht-erfindungsgemäße Verfahren ist dadurch gekennzeichnet, dass bei einer Temperatur inkubiert wird (Schritt b), Schritt d) und/oder Schritt e)), bei welcher der erste Teil der Liganden und der zweite Teil der Liganden in der Lösung zu Ligandenkomplexen hybridisiert sind.

In Gegenwart eines Rezeptors, der zu einer bestimmten Kombination aus zwei Liganden (bestimmter binärer Ligandenkomplex aus Ligand 1 und Ligand 2 über dsDNA oder dsRNA) eine hohe Affinität aufweist, bildet sich ein hochaffiner ternärer Liganden-Rezeptor-Komplex aus den zwei Liganden und dem Rezeptor. Hochaffinen Ligandenpaare werden somit aus dem Pool an binären Ligandenpaaren abgereichert und in der Form eines ternären Ligandenpaar-Rezeptor-Komplex angereichert.

Der Nachteil von Verfahren aus dem Stand der Technik ist, dass ein bestimmter Prozentsatz an hochaffinen Liganden mit wenig bis gar nicht affinen Liganden hybridisiert und somit in wenig affinen Ligandenkomplexen "gefangen" ist. Dieses Problem wird durch das nicht-erfindungsgemäße Verfahren gelöst. Durch einen Verfahrensschritt, in dem Ligandenpaare gezielt dissoziiert werden, werden die vorher im Ligandenkomplex "gefangenen" hochaffinen Liganden wieder "frei", um in einem weiteren Hybridisierungsschritt des Verfahren mit anderen freien hochaffinen Liganden zu neuen Ligandenkomplexen zu hybridisieren. Die neu gebildeten Ligandenkomplexe stehen wiederrum zur Bildung von Liganden-Rezeptor-Komplexen zur Verfügung. Im Ergebnis können somit gezielt die hochaffinen Liganden - im Idealfall quantitativ - in Liganden-Rezeptor-Komplexe überführt werden, was eine größere Menge an Liganden-Rezeptor-Komplexen bewirkt und somit die Sensitivität des nicht-erfindungsgemäßen Verfahrens gegenüber Verfahren aus dem Stand der Technik erhöht (verbessertes Signal-Stör-Verhältnis). Tatsächlich kann die Häufigkeit der Bildung des hochaffinen Ligandenpaares bei einer Anzahl von N theoretisch möglichen hochaffinen Ligandenpaaren in einer Bibliothek idealerweise bis zu einem Faktor N gesteigert werden (quantitative Bildung hochaffiner Komplexe, keine hochaffinen Liganden an niedrigaffine Liganden gebunden und damit "gefangen").

In dem nicht-erfindungsgemäßen Verfahren können die Ligandenkomplexe beispielsweise durch Erhöhung der Temperatur der Lösung dissoziiert werden, insbesondere auf eine Temperatur von 35 °C bis 95 °C, bevorzugt 50 °C bis 95 °C, besonders bevorzugt 70 °C bis 95 °C, insbesondere 80 °C bis 95 °C.

In dem nicht-erfindungsgemäßen Verfahren wird die Dissoziation der Ligandenkomplexe bevorzugt in einem Teil der Lösung durchgeführt, der von dem Rezeptor räumlich beabstandet ist, bevorzugt dergestalt, dass der Rezeptor durch die Bedingungen, die zur Dissoziation führen, funktional nicht beeinträchtigt wird. Der Vorteil an dieser Ausführungsform des nicht-erfindungsgemäßen Verfahrens ist, dass sich die Bewirkung der Dissoziation nicht negativ auf die (biologische) Funktion des Rezeptors auswirkt. Insbesondere werden hierdurch Rezeptoren vor thermischer und/oder pH-induzierter Denaturierung geschützt (z.B. Rezeptoren, die Proteine enthalten oder daraus bestehen).

In dem nicht-erfindungsgemäßen Verfahren können die freien Liganden (wieder) durch Erniedrigung der Temperatur der Lösung hybridisiert werden, insbesondere durch Erniedrigung auf eine Temperatur von 1 °C bis 30 °C, bevorzugt 5 °C bis 28 °C, besonders bevorzugt 10 °C bis 25 °C, insbesondere 15 bis 20 °C.

Die Schritte c) bis e) des nicht-erfindungsgemäßen Verfahrens können mindestens einmal oder zweimal, bevorzugt mindestens 3 oder 4 mal, besonders bevorzugt mindestens 5 oder 6 mal, insbesondere mindestens 7 oder 8 mal, wiederholt werden. Je öfter die Schritte wiederholt werden, desto mehr hochaffine Ligandenkomplexe werden schrittweise in den Liganden-Rezeptorkomplex überführt (Anstieg der Sensitivität).

In einer bevorzugten Ausführungsform des nicht-erfindungsgemäßen Verfahrens sind mindestens 20 Basen, bevorzugt mindestens 40 Basen, besonders bevorzugt mindestens 100 Basen, insbesondere mindestens 200 Basen, der einzelsträngigen DNA oder RNA von einem ersten Teil der Liganden zu der einzelsträngigen DNA oder RNA von einem zweiten Teil der Liganden komplementär.

Es wird ferner eine erfindungsgemäße Lösung zu der erfindungsgemäßen Aufgabe bereitgestellt, die ohne Aufheiz- und Abkühlschritte auskommt.

In dieser Hinsicht wird erfindungsgemäß ein Verfahren zur sensitiven Identifizierung hochaffiner Komplexe aus zwei Liganden und einem Rezeptor bereitgestellt, das die folgenden Schritte umfasst:
i) Interagieren von einer Vielzahl von verschiedenen Komplexen aus Liganden einer chemischen Bibliothek mit zumindest einem Rezeptor in einer Lösung, wobei die Liganden der Bibliothek eine einzelsträngige DNA oder RNA mit einer Basenlänge von mehr als 2 Basen aufweisen, die chemisch kovalent an die Liganden gebunden ist und wobei nur 2 bis 10 Basen der einzelsträngigen DNA oder RNA von einem ersten Teil der Liganden zu Basen der einzelsträngigen DNA oder RNA von einem zweiten Teil der Liganden komplementär sind;
ii) Inkubieren der Lösung für eine bestimmten Zeitraum, wobei Komplexe aus Ligandenkomplexen und dem Rezeptor entstehen; und
iii) Identifizieren der entstandenen Komplexe aus Ligandenkomplexen und dem Rezeptor.

Das Verfahren ist dadurch gekennzeichnet, dass die Lösung bei einer Raumtemperatur inkubiert wird, bei der sich zwischen dem ersten Teil der Liganden und dem zweiten Teil der Liganden ein Gleichgewicht aus Hybridisierung zu Ligandenkomplexen und Dissoziation zu freien Liganden einstellt (Raumtemperatur d.h. 15°C bis 30 °C). Dadurch wird eine dynamische Hybridisierung und Dissoziation erhalten. Ferner ist das Verfahren dadurch gekennzeichnet, dass in Schritt i) die Liganden über Hybridisierung der DNA oder RNA zu Ligandenkomplexen komplexiert sind, wobei die einzelsträngige DNA oder RNA bei dem ersten Teil der Liganden und die einzelsträngige DNA oder RNA bei dem zweiten Teil der Liganden eine Basensequenz enthält, die für den chemisch kovalent gebundenen Liganden kodiert.

Der Unterschied zu dem erstgenannten, nicht-erfindungsgemäßen Verfahren ist, dass durch die geringere Anzahl an komplementären Basen zwischen den beiden Teilen der Liganden eine Assoziation (über Hybridisierung) und Dissoziation der Liganden bereits bei Raumtemperatur stattfindet (dynamisches Gleichgewicht). Durch die Gegenwart von einem Rezeptor werden hochaffine Ligandenpaare dem dynamischen Gleichgewicht "entzogen" und in den Liganden-Rezeptor-Komplex überführt. Hochaffine Ligandenpaare werden also aus dem allgemeinen Ligandenpool der chemischen Bibliothek abgereichert und der hochaffine, ternäre Liganden-Rezeptor-Kompex angereichert. Durch das dynamische Gleichgewicht können daher auch ohne Aufheizen und Abkühlen alle hochaffinen Ligandenpaare (im Idealfall quantitativ) in den Rezeptorkomplex überführt werden, was die Konzentration von Ligand-Rezeptor-Komplexen erhöht und somit die Sensitivität der Detektionsmethode verbessert. Der Nachteil ist jedoch eine insgesamt etwas geringe Affinität der Ligandenpaare zum Rezeptor verglichen mit den Ligandenpaaren aus dem erstgenannten, nicht-erfindungsgemäßen Verfahren, da die Stabilität der Ligandenpaare durch die geringere Anzahl an komplementären Basen etwas geringer ist und somit im Gleichgewicht auch der ternäre Liganden-Rezeptor-Komplex weniger stark populiert ist.

In dieser erfindungsgemäßen Variante können nur 3 bis 9 Basen, bevorzugt nur 4 bis 8 Basen, besonders bevorzugt nur 5 bis 7 Basen, der einzelsträngigen DNA oder RNA von einem ersten Teil der Liganden zu der einzelsträngigen DNA oder RNA von einem zweiten Teil der Liganden komplementär sein.

In beiden Verfahren kann die Länge der DNA oder RNA von dem ersten Teil der Liganden und/oder zweiten Teil der Liganden mindestens 20 Basen, bevorzugt mindestens 40 Basen, besonders bevorzugt mindestens 100 Basen, insbesondere mindestens 200 Basen betragen.

Der erste und/oder zweite Teil der Liganden kann mehr als 20%, bevorzugt mehr als 30 %, besonders bevorzugt mehr als 40%, insbesondere 50%, der Gesamtmenge der Liganden ausmachen. Der erste Teil der Liganden weist bevorzugt L verschiedene Liganden und der zweite Teil der Liganden weist bevorzugt M verschiedene Liganden auf, sodass L·M verschiedene binäre Ligandenkomplexe gebildet werden. Für eine Bibliothek mit L·M binären Ligandenkomplexen kann ein bestimmter hochaffiner Ligandenkomplex mit einer erhöhten Effizienz von mindestens 1,5 bis N-fach, bevorzugt 2 bis N^{(1/2)}-fach, insbesondere 3 bis 10-fach, angereichert werden.

In einer bevorzugten Ausführungsform wird die Lösung in mindestens einem der Schritte b), e) und ii) bei einer Temperatur von 1 °C bis 50 °C, bevorzugt 5 °C bis 37 °C, besonders bevorzugt 10 °C bis 25 °C, insbesondere 15 bis 20 °C, inkubiert. Bei diesen Temperaturbereichen ist vorteilhaft, dass hier viele Rezeptortypen (insbesondere Proteinrezeptoren) stabil sind und ihre Funktion, Liganden zu binden, nicht verloren haben.

Die Lösung kann in mindestens einem der Schritte b), e) und ii) für einen Zeitraum von 0,1 bis 48 Stunden, bevorzugt 0,2 bis 24 Stunden, besonders bevorzugt 0,5 bis 12 Stunden, insbesondere 1 bis 6 Stunden, inkubiert werden. Längere Inkubationszeiten haben sich als vorteilhaft erwiesen, da die Bindung der Ligandenpaare an den Rezeptor zwar thermodynamisch begünstigt sein kann, aber dennoch eine langsame Kinetik aufweisen kann.

Der Rezeptor, der in den erfindungsgemäßen Verfahren eingesetzt wird kann immobilisiert sein, bevorzugt auf einem Substrat ausgewählt aus der Gruppe bestehend aus Glas, Keramik, Biopolymer, Sepharose, synthetisches Polymer und Hydrogel. Insbesondere kann hierbei die Lösung mit Liganden in einem Kreislauf an dem immobilisierten Rezeptor vorbeigeführt werden. Diese Ausführungsform ist vorteilhaft, wenn in dem Verfahren die Dissoziation der Ligandenkomplexe in einem Teil der Lösung durchgeführt wird, der von dem Rezeptor räumlich beabstandet ist, da hierbei Dissoziation und Hybridisierung der Liganden zwar räumlich getrennt, aber dennoch zeitgleich ablaufen können (z.B. Dissoziation in Rezeptorferne, Hybridisierung in Rezeptornähe).

Bevorzugt enthält der Rezeptor ein Protein, eine DNA, eine RNA, eine Zelle und/oder ein organisches Molekül mit einer molekularen Masse < 200 kDa, bevorzugt ≤ 100 kDa, besonders bevorzugt ≤ 10 kDa, insbesondere ≤ 3 kDa, oder besteht daraus.

Die eingesetzten Liganden können ein Molekül ausgewählt aus der Gruppe bestehend aus Protein, Peptid, Lipid, Kohlenhydrat, dsDNA, ssDNA, dsRNA und ssRNA, Aptamer, organisches Molekül mit einer molekularen Masse ≤ 200 kDa, bevorzugt <_ 100 kDa, besonders bevorzugt <_ 10 kDa, insbesondere < 3 kDa, enthalten oder daraus bestehen.

In einer bevorzugten Ausführungsform werden die Komplexe aus Ligandenkomplexen und dem Rezeptor über ein analytisches Verfahren identifiziert. Das Verfahren ist bevorzugt ausgewählt aus der Gruppe bestehend aus Massenspektrometrie, HPLC, Gaschromatographie, IR-Spektroskopie und DNA-Sequenzierung, bevorzugt DNA-Sequenzierung eines DNA- oder RNA-Barcodes.

Erfindungsgemäß enthält die einzelsträngige DNA oder RNA bei dem ersten und zweiten Teil der Liganden eine Basensequenz, die für den chemisch kovalent gebundenen Liganden kodiert (Basensequenz als Barcode), wobei diese Basensequenz bevorzugt zu einer komplementären Basensequenz einer weiteren einzelsträngigen DNA oder RNA hybridisiert ist. Der Vorteil an dem Barcode liegt in der einfachen Identifizierung von komplexgebundenen Liganden über DNA- bzw. RNA-Sequenzierung. Besonders vorteilhaft ist, wenn die Basensequenz der Liganden eine komplementäre einzelsträngige DNA- oder RNA hybridisiert enthält, die ebenfalls als Barcode dienen kann. Enthalten beide Liganden des Liganden-Rezeptor-Komplexes (Ligand 1 und 2) eine einzelsträngige DNA bzw. RNA, so kann diese z.B. durch Zugabe einer Ligase ligiert werden, wobei eine einzelsträngige DNA- oder RNA erhalten wird, die für die spezifische Kombination von Ligand 1 und 2 kodiert Der (ligierte) Barcode kodiert in diesem Fall also nicht nur für nur einen einzelnen Liganden, sondern für zwei Liganden, die zusammen einen hochaffinen Ligandenkomplex bilden.

Die in dem Verfahren eingesetzte einzelsträngige
i) DNA kann Adenin, Thymin, Guanin und/oder Cytosin; und/oder
ii) RNA kann Adenin, Uracil, Guanin, Cytosin und/oder Inosin;
enthalten.

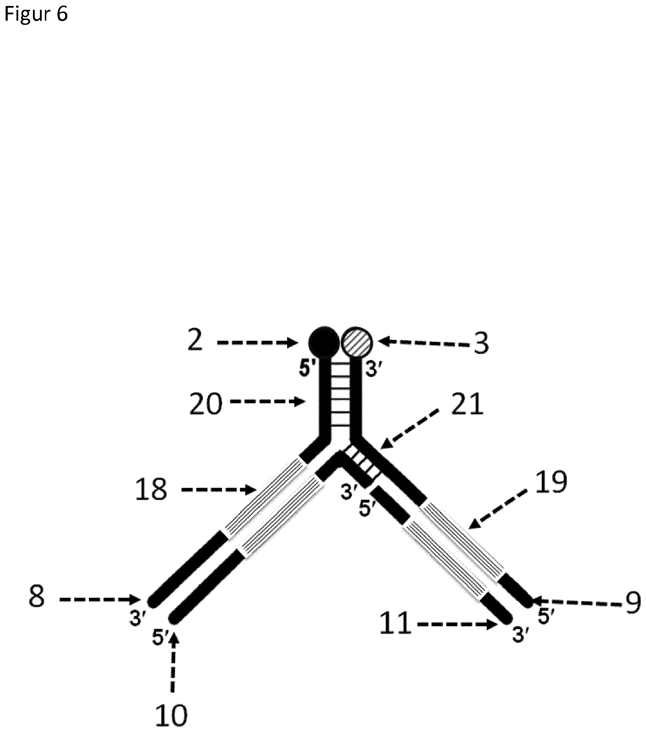

Das Verfahren ist in einer vorteilhaften Ausführungsform dadurch gekennzeichnet, dass die einzelsträngige DNA oder RNA der Liganden der Bibliothek bereichsweise zu einer komplementären Basensequenz einer weiteren einzelsträngigen DNA oder RNA hybridisiert ist, wobei 2 bis 10 Basen der weiteren einzelsträngigen DNA oder RNA von einem ersten Teil der Liganden zu Basen der einzelsträngigen DNA oder RNA von einem zweiten Teil der Liganden komplementär sind und während dem Verfahren an die komplementären Basen binden (bevorzugt unter Ausbildung einer Y-Form, insbesondere wie in Fig. 6 dargestellt), wobei während dem Verfahren eine Ligase zugegeben wird, welche die weitere einzelsträngige DNA oder RNA von dem ersten Teil der Liganden mit der weiteren einzelsträngigen DNA oder RNA von dem zweiten Teil der Liganden chemisch kovalent ligiert.

Eine Ligation der beiden weiteren DNAs oder RNAs findet somit nur dann statt, falls es zur Hybridisierung der jeweiligen Basen kommt d.h. falls der Gesamtkomplex hinreichend stabil ist. Vorteilhaft ist an dieser Ausführungsform jedoch, dass eine einmal stattgefundene Komplexbildung durch die chemisch kovalente Ligation der beiden weiteren DNAs bzw. RNAs "gespeichert" wird. Anders ausgedrückt akkumulieren die ligierten weiteren DNAs bzw. RNAs während des Verfahrens, falls die Grundlage für ihre Ligation eine stabile Bildung des Gesamtkomplexes war. Auf diesem Weg kann eine deutliche Steigerung der Sensitivität des Verfahrens erreicht werden, zumal die akkumulierte, ligierte DNA bzw. RNA über bekannte Verfahren (PCT, RT-PCR) weiter amplifiziert werden kann. Folglich können auf diese Art und Weise auch sehr geringe Konzentrationen an Liganden eingesetzt werden bzw. auch bei sehr geringen Rezeptorkonzentrationen hochaffine Liganden identifiziert werden.

Bei der oben aufgeführten Ausführungsform können bei einer einzelsträngigen DNA oder RNA von einem ersten Teil der Liganden,
i) die den Ligand an ihrem 5'-Ende gebunden hat, die Basen, die zu den Basen der einzelsträngigen DNA oder RNA von einem zweiten Teil der Liganden komplementär sind, an ihrem 5'-Ende aufweisen und die 2 bis 10 Basen der weiteren einzelsträngigen DNA oder RNA, die zu Basen der einzelsträngigen DNA oder RNA von einem zweiten Teil der Liganden komplementär sind, am 3'-Ende der weiteren einzelsträngigen DNA oder RNA angeordnet sein; oder
ii) die den Ligand an ihrem 3'-Ende gebunden hat, die Basen, die zu den Basen der einzelsträngigen DNA oder RNA von einem zweiten Teil der Liganden komplementär sind, an ihrem 3'-Ende aufweisen und die 2 bis 10 Basen der weiteren einzelsträngigen DNA oder RNA, die zu Basen der einzelsträngigen DNA oder RNA von einem zweiten Teil der Liganden komplementär sind, am 5'-Ende der weiteren einzelsträngigen DNA oder RNA angeordnet sein.

Durch diese spezifische Anordnung der jeweiligen komplementären Basen wird die Ausbildung einer Y-Struktur begünstigt, was wiederrum die Effizienz der Ligation der beiden weiteren einzelsträngigen DNAs oder RNAs durch die Ligase steigert.

Ferner wird eine mikrofluidische Vorrichtung zur Durchführung des erfindungsgemäßen Verfahrens bereitgestellt. Die Vorrichtung umfasst:
a) einen Behälter zur Aufnahme von immobilisiertem Rezeptor, wobei der Behälter einen Zugang mit Ventil aufweist, der zur Injektion von Liganden einer Bibliothek und zur Isolierung von Ligand-Rezeptor-Komplexen dient und der Behälter einen Eingang und eine Ausgang zu einer fluidischen Leitung aufweist;
b) eine fluidische Leitung, die mit einem Eingang und einem Ausgang des Behälters verbunden ist und die an einer Stelle einen Ausgang mit Ventil aufweist;
c) eine Heizvorrichtung, die in einem ersten Bereich der fluidischen Leitung angeordnet ist; und
d) einen Kühlbereich, der sich in einem zweiten, vom ersten verschiedenen, Bereich der fluidischen Leitung befindet.

In der mikrofluidischen Vorrichtung kann die Erhitzung von in Lösung befindlichen Liganden und die Selektion von Liganden, die an immobilisierten Rezeptor gebunden sind, lokal voneinander getrennt stattfinden und somit eine thermische Schädigung des Rezeptors (z.B. Proteindenaturierung) verhindert werden. Der Behälter kann hierbei immobilisierten Rezeptor zurückhalten und verhindern, dass immobilisierter Rezeptor in die fluidischen Leitungen gerät. Bevorzugt weist der Behälter somit Filter auf, die ein Passieren von immobilisiertem Rezeptor in die fluidische Leitung verhindern.

In einer bevorzugten Ausgestaltungsform weist der Kühlbereich der Vorrichtung eine Kühlvorrichtung auf und/oder ist in dem Bereich des Ausgangs mit Ventil angeordnet.

Die Vorrichtung kann eine Pumpe aufweisen, die so angeordnet ist, dass sie eine Flüssigkeit durch die fluidische Leitung pumpen kann. Insbesondere kann der Behälter der Vorrichtung Filter aufweisen, die ein Passieren von immobilisiertem Rezeptor in die fluidische Leitung verhindern.

Die mikrofluidische Vorrichtung kann dadurch gekennzeichnet sein, dass der Behälter und/oder die fluidische Leitung eine erfindungsgemäße dynamische, selbst-assemblierende chemische Bibliothek enthält (s.u.).

Es wird ferner eine chemische Bibliothek, bereitgestellt, die Liganden enthält, die eine einzelsträngige DNA oder RNA mit einer Basenlänge von mehr als 2 Basen aufweisen, die chemisch kovalent an einen Liganden gebunden sind, wobei 2 bis 10 Basen der einzelsträngigen DNA oder RNA von einem ersten Teil der Liganden zu Basen der einzelsträngigen DNA oder RNA von einem zweiten Teil der Liganden komplementär sind. Die chemische Bibliothek ist dadurch gekennzeichnet, dass sie binäre Komplexe der jeweiligen Liganden enthält, wobei die einzelsträngige DNA oder RNA bei dem ersten Teil der Liganden und die einzelsträngige DNA oder RNA bei dem zweiten Teil der Liganden eine Basensequenz enthält, die für den chemisch kovalent gebundenen Liganden kodiert.

Es können hierbei nur 3 bis 9 Basen, bevorzugt nur 4 bis 8 Basen, besonders bevorzugt nur 5 bis 7 Basen, der einzelsträngigen DNA oder RNA von einem ersten Teil der Liganden zu der einzelsträngigen DNA oder RNA von einem zweiten Teil der Liganden komplementär ein.

Die Länge der DNA oder RNA von dem ersten Teil der Liganden und/oder zweiten Teil der Liganden kann mindestens 20 Basen, bevorzugt mindestens 40 Basen, besonders bevorzugt mindestens 100 Basen, insbesondere mindestens 200 Basen betragen.

In einer bevorzugten Ausgestaltungsform macht der erste und/oder zweite Teil der Liganden mehr als 20%, bevorzugt mehr als 30 %, besonders bevorzugt mehr als 40%, insbesondere 50%, der Gesamtmenge der Liganden der Bibliothek aus. Der erste Teil der Liganden L weist bevorzugt L verschiedene Liganden und der zweite Teil der Liganden weist bevorzugt L verschiedene Liganden auf, sodass die Bibliothek L² verschiedene Ligandenkomplexe aufweist.

Die Liganden der Bibliothek können ein Molekül ausgewählt aus der Gruppe bestehend aus Protein, Peptid, Lipid, Kohlenhydrat, dsDNA, ssDNA, dsRNA und ssRNA, Aptamer, organisches Molekül mit einer molekularen Masse ≤ 200 kDa, bevorzugt ≤ 100 kDa, besonders bevorzugt ≤ 10 kDa, insbesondere ≤ 3 kDa, enthalten oder daraus bestehen.

Die einzelsträngige
i) DNA kann Adenin, Thymin, Guanin und/oder Cytosin; und/oder
ii) RNA kann Adenin, Uracil, Guanin, Cytosin und/oder Inosin;
enthalten.

Die Bibliothek kann dadurch gekennzeichnet sein, dass die einzelsträngige DNA oder RNA der Liganden der Bibliothek jeweils bereichsweise zu einer komplementären Basensequenz einer weiteren einzelsträngigen DNA oder RNA hybridisiert ist, wobei 2 bis 10 Basen einer ersten weiteren einzelsträngigen DNA oder RNA von einem ersten Teil der Liganden zu Basen der zweiten einzelsträngigen DNA oder RNA von einem zweiten Teil der Liganden komplementär sind.

Die einzelsträngige DNA oder RNA von einem ersten Teil der Liganden,
i) die den Ligand an ihrem 5'-Ende aufweist, kann die Basen, die zu den Basen der einzelsträngigen DNA oder RNA von einem zweiten Teil der Liganden komplementär sind, an ihrem 5'-Ende aufweisen und die 2 bis 10 Basen der weiteren einzelsträngigen DNA oder RNA, die zu Basen der zweiten einzelsträngigen DNA oder RNA von einem zweiten Teil der Liganden komplementär sind, am 3'-Ende der weiteren einzelsträngigen DNA oder RNA angeordnet sein; oder
ii) die den Ligand an ihrem 3'-Ende aufweist, kann die Basen, die zu den Basen der einzelsträngigen DNA oder RNA von einem zweiten Teil der komplementär sind, an ihrem 3'-Ende aufweisen und die 2 bis 10 Basen der weiteren einzelsträngigen DNA oder RNA, die zu Basen der einzelsträngigen DNA (9) oder RNA von einem zweiten Teil der Liganden komplementär sind, am 5'-Ende der weiteren einzelsträngigen DNA oder RNA angeordnet sein.

Letztlich wird die Verwendung der erfindungsgemäßen chemischen Bibliothek zur selektiven und sensitiven Identifizierung von hochaffinen ternären Liganden-Rezeptor-Komplexen vorgeschlagen.

Anhand der nachfolgenden Figuren soll der erfindungsgemäße Gegenstand näher erläutert werden, ohne diesen auf die hier dargestellten spezifischen Ausführungsformen einschränken zu wollen.

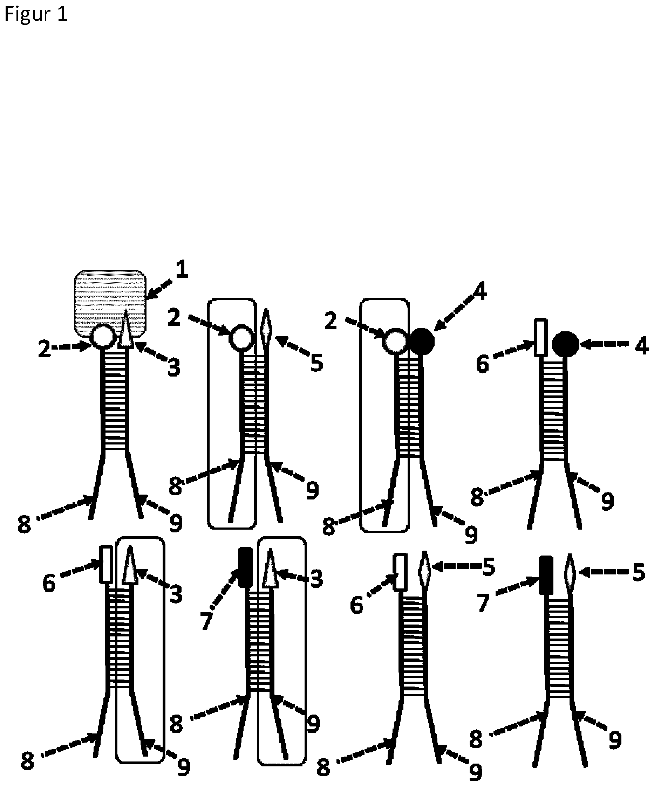

Von den folgenden Ausführungsformen zeigen die Figuren 1, 3 und 4 keine Ausführungsformen im Sinne der Erfindung, sind aber hilfreich, um bestimmte Aspekte der Erfindung zu verstehen.

Figur 1 zeigt eine Vielzahl von verschiedenen Liganden 2, 3, 4, 5, 6, 7 einer chemischen Bibliothek und zumindest einen Rezeptor 1. Die Liganden 2, 3, 4, 5, 6, 7 sind jeweils an eine einzelsträngige DNA 8, 9 chemisch kovalent gebunden, wobei die einzelsträngige DNA 8, 9 eine Basenlänge von mehr als 10 Basen aufweist. Die einzelsträngige DNA 8, 9 kodiert jeweils für den jeweiligen Liganden, an den sie gebunden ist, über ihre Basensequenz. In diesem nicht-erfindungsgemäßen Beispiel sind 18 Basen der einzelsträngigen DNA 8 von einem ersten Teil der Liganden 2, 6, 7 zu Basen der einzelsträngigen DNA 9 von einem zweiten Teil der Liganden 3, 4, 5 komplementär (siehe gestrichelte Linie zwischen den einzelsträngigen DNAs 8, 9). Hier bindet nur ein bestimmtes Ligandenpaar (gebildet durch die Liganden der Bezugsteichen 2 und 3) mit hoher Affinität an den Rezeptor 1. Andere Ligandenpaare (gebildet durch die Liganden der Bezugszeichen 4, 5, 6, 7) binden schwach bis gar nicht an den Rezeptor 1. Durch die hohe Anzahl an komplementären Basen zwischen den einzelsträngigen DNAs 8, 9 von zwei Liganden 2, 3, 4, 5, 6, 7 werden die Ligandenkomplexe stabilisiert, was bei ihrem Einsatz in einem Detektionsverfahren eine hohe Sensitivität bewirkt. Um eine Dynamik der Hybridisierung und Dissoziation zu erreichen ist ein permanentes Aufheizen (Dissoziation) und Abkühlen (Reassoziation bzw. Hybridisierung) d.h. eine Energiezufuhr nötig.

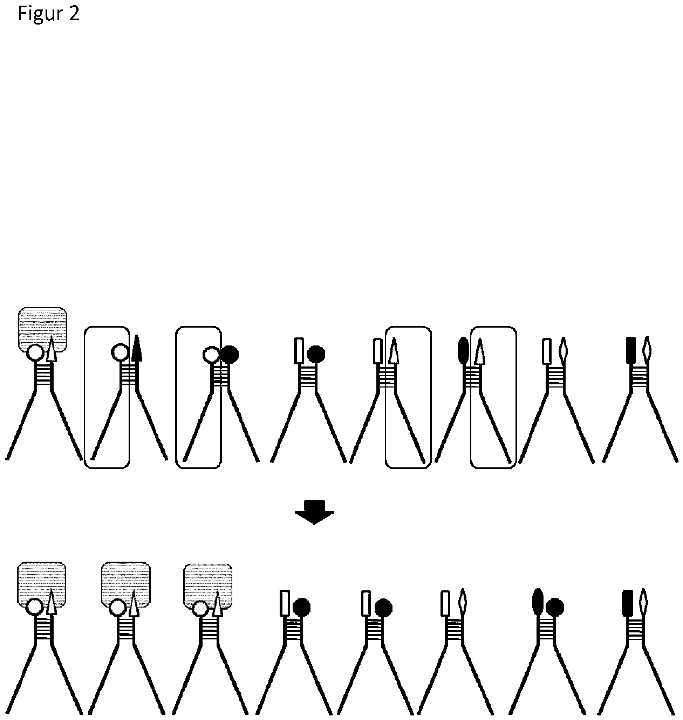

Figur 2 zeigt eine zu Figur 1 entsprechende Abbildung mit dem Unterschied, dass in diesem Ausführungsbeispiel nur 6 Basen der einzelsträngigen DNA von einem ersten Teil der Liganden zu Basen der einzelsträngigen DNA oder RNA von einem zweiten Teil der Liganden komplementär sind (sonst sind die Moleküle und Bezeichnungen zu Figur 1 identisch). Durch die niedrigere Zahl an komplementären Basen ergibt sich der Vorteil, dass die Assoziation (Hybridisierung) von zwei Liganden schon bei Raumtemperatur (15°C bis 30 °C) hochdynamisch ist d.h. eine permanente Hybridisierung und Dissoziation stattfindet. Die bei bestimmten Ligandenpaaren vorliegende hochaffine Bindung an Rezeptor hemmt die Dissoziation dieser Ligandenpaare, wodurch Komplexe aus Rezeptor und hochaffinen Ligandenpaaren länger populiert sind, im Lauf der Zeit "akkumulieren" und letztlich im Gleichgewicht stärker populiert sind. Vorteilhaft an dieser Ausführungsform ist, dass die Dynamik der Assoziation und Hybridisierung bei Raumtemperatur stattfindet d.h. im Gegensatz zur nicht-erfindungsgemäßen Ausführungsform der Figur 1 keine thermische Energie zugeführt werden muss. Der Nachteil an dieser Ausführungsform ist eine niedrigere Sensitivität als bei der nicht-erfindungsgemäßen Ausführungsform in Figur 1, da die geringere Anzahl an Basenpaaren (6 statt 18) den Ligandenkomplex und damit den Komplex der Liganden mit dem Rezeptor instabiler macht, wodurch dieser im Gleichgewicht weniger stark populiert ist als in der Ausführungsform in Figur 1.

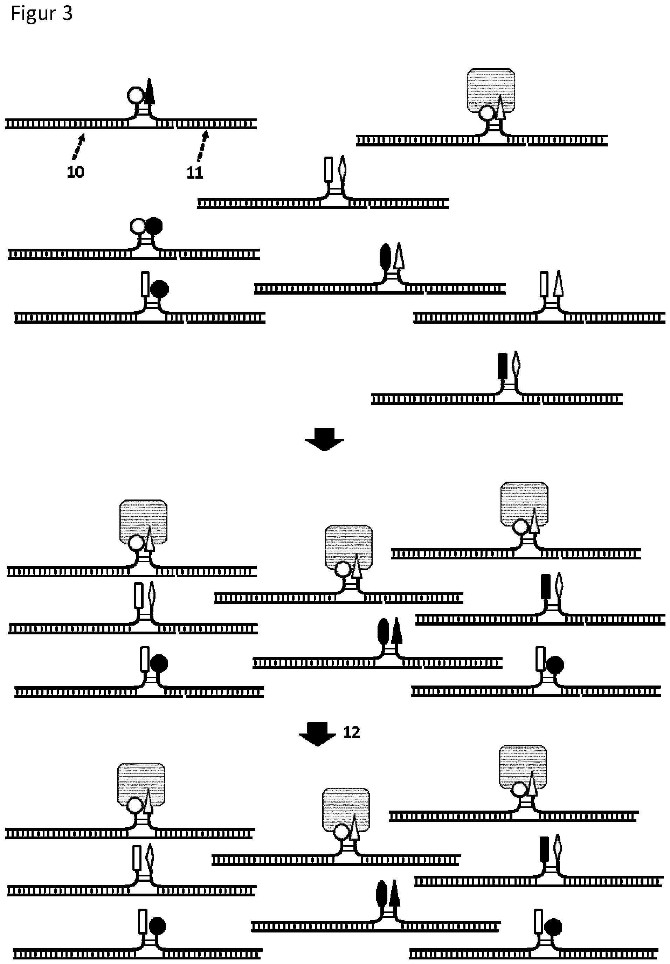

Figur 3 verdeutlicht über eine Reaktionsgleichung wie die hochaffinen Ligandenpaare als Komplex mit dem Rezeptor im Lauf der Zeit "akkumulieren" (siehe erster Pfeil oben). Zusätzlich zu den bereits aus Figur 1 und Figur 2 genannten Molekülen weisen die Ligandenpaare noch eine jeweils zur an den Liganden kovalent gebundenen einzelsträngigen DNA eine komplementäre einzelsträngige DNA 10, 11 auf (sonst sind die Moleküle und Bezeichnungen zu Figur 1 identisch). Diese zusätzliche einzelsträngige DNA 10, 11 kann beispielsweise über geeignete Primer und eine PCR-Reaktion generiert werden. Der untere Pfeil 12 symbolisiert eine Ligationsreaktion (z.B. durch Zugabe eines Ligaseenzyms), die dazu führt, dass die beiden zusätzlichen einzelsträngigen DNAs 10, 11 chemisch kovalent aneinander ligiert werden. Der Vorteil hierbei ist, dass das ligierte DNA-Stück jeweils über seine Basensequenz für ein spezifisches Ligandenpaar kodiert, welches über DNA-Sequenzierung somit leicht identifiziert werden kann.

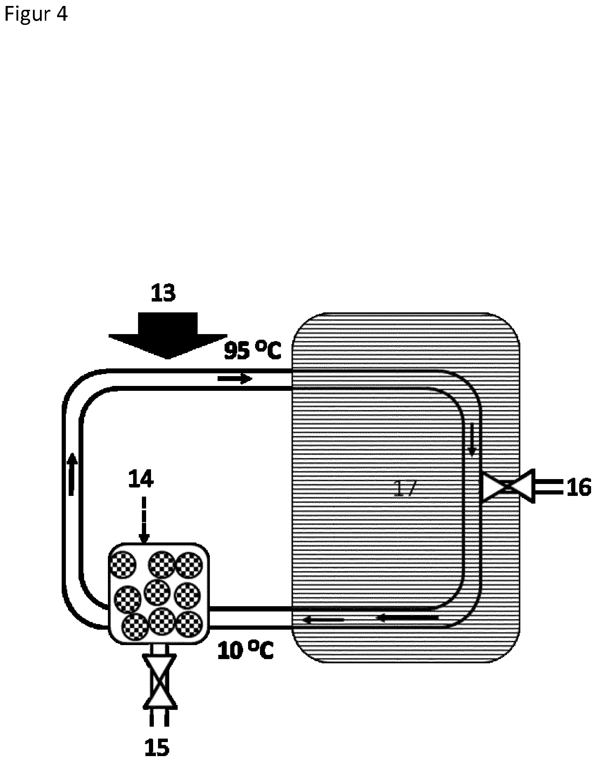

Figur 4 beschreibt eine mikrofluidische Vorrichtung. In einem Behälter 14 befindet sich immobilisierter Rezeptor, der über die Öffnung 15 mit Ventil in den Behälter gefüllt wurde. Der Behälter 14 ist auf der einen Seite und der anderen Seite mit einer Leitung verbunden, durch die eine Flüssigkeit geführt werden kann. Die Leitung ist in einer Zone, die beheizt werden kann. Eine Beheizung kann über einen IR-Strahler als Heizquelle 13 erfolgen. Die Vorrichtung weist ferner und eine Kühlungszone 17 auf. Optional enthält diese Zone eine Kühlvorrichtung. Zudem weist die Leitung, hier im Kühlbereich 17, einen Ausgang 16 mit einem Ventil auf, aus dem Flüssigkeit mit (freien) Liganden entnommen werden kann und einer Analyse zugeführt werden kann. Zudem kann nach einer bestimmten Inkubationszeit über die Öffnung 15 mit hochaffinen Liganden beladener immobilisierter Rezeptor entnommen werden.

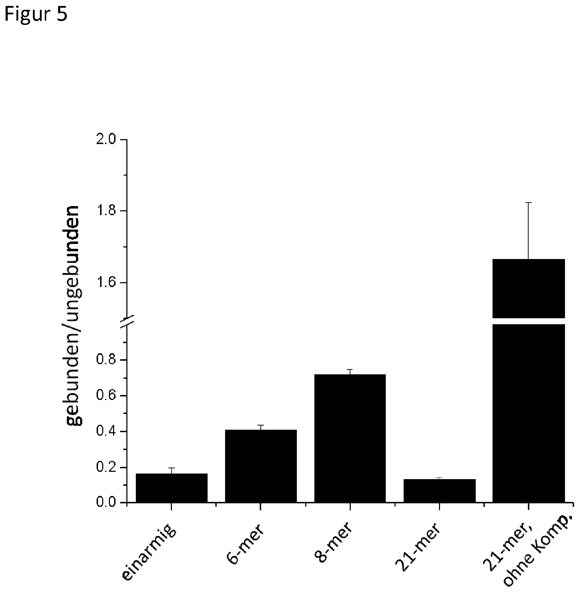

Figur 5 zeigt das Ergebnis von einem Experiment, welches die Wirksamkeit des erfindungsgemäßen Verfahrens belegt. Es wurde als Ligand Iminobiotin verwendet, das jeweils an ssDNA chemisch kovalent gebunden war (= Im-ssDNA). Die Im-ssDNA war zu gleichen Teilen aufgeteilt in einen ersten Teil an Im-ssDNA1 und einen zweiten Teil an Im-ssDNA2, wobei Im-ssDNA1 und Im-ssDNA2 je nach Experiment eine unterschiedliche Anzahl zueinander komplementärer Basen aufwiesen (z.B. 6 komplementäre Basen bei "6-mer"). Im-ssDNA1 wurde chemisch kovalent an den Fluoreszenzfarbstoff Cy5 gekoppelt, um auf der einen Seite eine Detektion und Quantifizierung von freier Im-ssDNA 1 bzw. dem freien binären Komplex aus Im-ssDNA1 und Im-ssDNA2 ohne Rezeptor und auf der anderen Seite die Detektion des ternären Im-ssDNA1·lm-ssDNA2·Rezeptor Komplexes zu ermöglichen. Als Rezeptor wurde immobilisiertes Streptavidin und als Lösung wurde ein wässriger Puffer mit pH 9,2 verwendet. Die Figur zeigt das Verhältnis der Menge an Liganden-Rezeptor-Komplex zu nicht an Rezeptor gebundenen Liganden ("gebunden/ungebunden" auf der y-Achse) unter kompetitiven Verhältnissen, d.h. unter Anwesenheit von Iminobiotin-freier ssDNA (ssDNA) als Beispiel für einen unaffinen Liganden (siehe "einarmig", "6-mer", "8-mer" und "21-mer" auf der x-Achse) bzw. nicht-kompetitiven Verhältnissen, d.h. ohne Anwesenheit kompetitiver ssDNA ("21mer, keine Komp." auf der x-Achse).

Außer bei dem nicht-kompetitiven Experiment ("21mer, keine Komp." auf der x-Achse) lag die Iminobiotin-freie ssDNA im 300-fachen Überschuss in der Lösung vor. In dem Experiment mit der Bezeichnung "einarmig" (auch ein 6-mer) war nur Im-ssDNA1, also keine Im-ssDNA2 anwesend, sodass sich keine binären Ligandenkomplexe bilden konnten. Das Experiment "einarmig" zeigt somit das Bindungsverhältnis für ein monomeres Iminobiotin zu Streptavidin auf. Im direkten Vergleich mit dem Experiment "6-mer" wird deutlich, dass unter den getesteten kompetitiven Bedingungen bei dimerem Iminobiotin (= binärer Ligandenkomplex) das Bindungsgleichgewicht deutlich in Richtung Liganden-Rezeptor-Komplex verschoben ist. Dieser Effekt ist durch die höhere Stabilisierung durch die Hybridisierung von 8 Basenpaaren bei dem Experiment "8-mer" noch deutlicher ausgeprägt. Steigt die Anzahl an komplementären Basen jedoch weiter an (z.B. hier 21 komplementäre Basen beim Experiment "21-mer"), so fällt die Menge an erhaltenen Liganden-Rezeptor-Komplex auf einen Wert, der ungefähr dem Wert des Experiments "einarmig" entspricht.

Diese Beobachtung lässt sich dadurch erklären, dass die Cy5-gelabelte Iminobiotin-gebundene ssDNA (Im-ssDNA1) in wenig affinen binären Komplexen mit Iminobiotin-freier ssDNA (ssDNA) "gefangen" wird und daher nicht mehr an komplementäre Cy5-freie, aber Iminobiotin-haltige ssDNA (Im-ssDNA2) binden kann. Dieser Effekt wird deshalb nur beim Experiment "21mer" beobachtet, da hier ein gebildeter lm-ssDNA1·ssDNA Komplex so stabil ist, dass bei Raumtemperatur (ohne weitere Energiezufuhr) praktisch keine Dissoziation dieses Komplexes mehr stattfindet. Bei einer Anzahl an komplementären Basen von 6 und 8 Basen ("6-mer" bzw. "8-mer") tritt dieser Effekt jedoch nicht auf nicht auf, da hier die Bildung des lm-ssDNA1·ssDNA Komplexes nicht statisch, sondern dynamisch ist und somit bei dieser geringeren Anzahl an komplementärer Basen eine durch ssDNA "blockierte" Iminobiotin-gebundene ssDNA (Im-ssDNA1) wieder frei wird und mit einer weiteren Iminobiotingebundenen ssDNA (Im-ssDNA2) zu einem hochaffinen binären Komplex reagieren kann. Folglich wird bei einer Anzahl an Basenpaaren von 6 und 8 bei Raumtemperatur eine "Akkumulation" von Komplexen aus Rezeptor (hier Streptavidin) und hochaffinen Ligandenpaaren (hier ein Paar aus zwei Iminobiotin-Molekülen) erreicht, wodurch dieses Verfahren im Hinblick auf die Sensitivität einem statischen Verfahren überlegen ist. Gesteigert werden kann die Sensitivität dadurch, dass eine Anzahl an 21 Basenpaaren verwendet wird und im Wechsel aufgeheizt (Dissoziation) und abgekühlt (Hybridisierung) wird d.h. Energie zugeführt wird.

Figur 6 zeigt eine Y-Form, die der Gesamtkomplex in einer bevorzugten Ausführungsform der Erfindung einnimmt. Der erste Ligand 2, der an die erste einzelsträngige DNA 8 chemisch kovalent gebunden ist, weist einen Abschnitt 18 auf, der für den ersten Ligand 2 kodiert. Der zweite Ligand 3, der an die zweite einzelsträngige DNA 9 chemisch kovalent gebunden ist, weist einen Abschnitt 19 auf, der für den zweiten Ligand 3 kodiert. Hier sind sechs Basen der ersten einzelsträngigen DNA 8 zu sechs Basen der zweiten einzelsträngigen DNA 9 komplementär und können während des Verfahrens Basenpaare 20 ausbilden (siehe die Linien zwischen den einzelsträngigen DNAs 8, 9). Ferner ist in diesem Ausführungsbeispiel die erste einzelsträngige DNA 8 bereichsweise (hier: am 3'-Ende) mit einer ersten zusätzlichen einzelsträngigen DNA 10 hybridisiert. Die erste zusätzliche einzelsträngige DNA 10 ist dadurch gekennzeichnet, dass sie einen "Überhang" von zwei bis zwölf Basen enthält (hier: fünf Basen, lokalisiert an ihrem 3'-Ende), die zu zwei bis zwölf Basen der zweiten einzelsträngigen DNA 9 komplementär sind (hier: fünf Basen) und die mit den komplementären Basen während des Verfahrens Basenpaare 20 ausbilden können (siehe die Linien zwischen der zusätzlichen ersten einzelsträngigen DNA 10 und der zweiten einzelsträngigen DNA 9).

Ist der zweite Ligand 3 über die zweite einzelsträngige DNA 9 bereichsweise mit einer geeigneten zweiten zusätzlichen DNA 11 hybridisiert, so bewirkt die Bildung des Gesamtkomplexes (hier: in Y-Form), dass die erste zusätzliche DNA 10 mit der zweiten zusätzlichen DNA 11 dergestalt zusammengeführt wird, dass sie über Zugabe eines Ligase-Enzyms chemisch kovalent ligiert werden können. Wird in dem Verfahren ein Ligase-Enzym zugegeben, so wird damit erreicht, dass Ligationsprodukte der beiden einzelsträngigen zusätzlichen DNAs 10, 11, die für hochaffine Ligandenkomplexe kodieren, im Laufe des Verfahrens akkumulieren. Dadurch steigt die Sensitivität deren Detektion. Da es sich bei dem Akkumulationsprodukt um DNA handelt, kann diese noch weiter amplifiziert werden (z.B. durch PCR), wodurch die Sensitivität des Verfahrens nochmals gesteigert wird. Zudem erlaubt eine Sequenzierung der ligierten DNA einen schnellen Rückschluss auf die beiden Liganden 2, 3, da die ligierte DNA die Abschnitte 18, 19 aufweist, die für beide Liganden 2, 3 kodiert.

## Patentansprüche

1. Verfahren zur sensitiven Identifizierung hochaffiner Komplexe aus zwei Liganden (2, 3, 4, 5, 6, 7) und einem Rezeptor (1), umfassend die Schritte
i) Interagieren von einer Vielzahl von verschiedenen Komplexen aus Liganden (2, 3, 4, 5, 6, 7) einer chemischen Bibliothek mit zumindest einem Rezeptor (1) in einer Lösung, wobei die Liganden (2, 3, 4, 5, 6, 7) der Bibliothek eine einzelsträngige DNA (8, 9) oder RNA mit einer Basenlänge von mehr als 2 Basen aufweisen, die chemisch kovalent an die Liganden (2, 3, 4, 5, 6, 7) gebunden ist und wobei nur 2 bis 10 Basen der einzelsträngigen DNA (8) oder RNA von einem ersten Teil der Liganden (2, 6, 7) zu Basen der einzelsträngigen DNA (9) oder RNA von einem zweiten Teil der Liganden (3, 4, 5) komplementär sind;
ii) Inkubieren der Lösung für einen bestimmten Zeitraum, wobei Komplexe aus Ligandenkomplexen und dem Rezeptor (1) entstehen;
iii) Identifizieren der entstandenen Komplexe aus Ligandenkomplexen und dem Rezeptor (1),
**dadurch gekennzeichnet, dass** die Lösung bei einer Raumtemperatur inkubiert wird, bei der sich zwischen dem ersten Teil der Liganden (2, 6, 7) und dem zweiten Teil der Liganden (3, 4, 5) ein Gleichgewicht aus Hybridisierung zu Ligandenkomplexen und Dissoziation zu freien Liganden (2, 3, 4, 5, 6, 7) einstellt, wobei in Schritt i) die Liganden über Hybridisierung der DNA (8, 9) oder RNA zu Ligandenkomplexen komplexiert sind, wobei die einzelsträngige DNA (8) oder RNA bei dem ersten Teil der Liganden (2, 6, 7) und die einzelsträngige DNA (9) oder RNA bei dem zweiten Teil der Liganden (3, 4, 5) eine Basensequenz enthält, die für den chemisch kovalent gebundenen Liganden (2, 3, 4, 5, 6, 7) kodiert.

2. Verfahren gemäß dem vorhergehenden Anspruch, **dadurch gekennzeichnet, dass** nur 3 bis 9 Basen, bevorzugt nur 5 bis 9 Basen, besonders bevorzugt nur 6 bis 8 Basen, der einzelsträngigen DNA (8) oder RNA von einem ersten Teil der Liganden (2, 6, 7) zu der einzelsträngigen DNA (9) oder RNA von einem zweiten Teil der Liganden (3, 4, 5) komplementär sind.

3. Verfahren gemäß einem der vorhergehenden Ansprüche, **dadurch gekennzeichnet, dass** die Lösung in Schritt ii)
a) bei einer Temperatur von 1 °C bis 50 °C, bevorzugt 5 °C bis 37 °C, besonders bevorzugt 10 °C bis 25 °C, insbesondere 15 bis 20 °C, inkubiert wird; und/oder
b) für einen Zeitraum von 0,1 bis 48 Stunden, bevorzugt 0,2 bis 24 Stunden, besonders bevorzugt 0,5 bis 12 Stunden, insbesondere 1 bis 6 Stunden, inkubiert wird.

4. Verfahren gemäß einem der vorhergehenden Ansprüche, **dadurch gekennzeichnet, dass** der Rezeptor (1)
i) immobilisiert ist, bevorzugt auf einem Substrat ausgewählt aus der Gruppe bestehend aus Glas, Keramik, Biopolymer, Sepharose, synthetisches Polymer und Hydrogel; und/oder
ii) ein Protein, eine DNA, eine RNA, eine Zelle und/oder ein organisches Molekül mit einer molekularen Masse ≤ 200 kDa, bevorzugt ≤ 100 kDa, besonders bevorzugt ≤ 10 kDa, insbesondere ≤ 3 kDa, enthält oder daraus besteht.

5. Verfahren gemäß einem der vorhergehenden Ansprüche, **dadurch gekennzeichnet, dass** die Liganden (2, 3, 4, 5, 6, 7) ein Molekül ausgewählt aus der Gruppe bestehend aus Protein, Peptid, Lipid, Kohlenhydrat, dsDNA, ssDNA, dsRNA und ssRNA, Aptamer, organisches Molekül mit einer molekularen Masse ≤ 200 kDa, bevorzugt ≤ 100 kDa, besonders bevorzugt ≤ 10 kDa, insbesondere ≤ 3 kDa, enthalten oder daraus bestehen.

6. Verfahren gemäß einem der vorhergehenden Ansprüche, **dadurch gekennzeichnet, dass** die Komplexe aus Ligandenkomplexen und dem Rezeptor (1) identifiziert werden über ein analytisches Verfahren, bevorzugt ausgewählt aus der Gruppe bestehend aus Massenspektrometrie, HPLC, Gaschromatographie, IR-Spektroskopie und DNA-Sequenzierung, insbesondere über DNA-Sequenzierung eines DNA- oder RNA-Barcodes.

7. Verfahren gemäß einem der vorhergehenden Ansprüche, **dadurch gekennzeichnet, dass** die Basensequenz, die für den chemisch kovalent gebundenen Liganden kodiert, zumindest bereichsweise zu einer komplementären Basensequenz einer weiteren einzelsträngigen DNA oder RNA hybridisiert ist.

8. Verfahren gemäß einem der vorhergehenden Ansprüche, **dadurch gekennzeichnet, dass** die einzelsträngige DNA (8, 9) oder RNA der Liganden (2, 3, 4, 5, 6, 7) der Bibliothek bereichsweise zu einer komplementären Basensequenz einer weiteren einzelsträngigen DNA (10, 11) oder RNA hybridisiert ist, wobei 2 bis 10 Basen der weiteren einzelsträngigen DNA (10) oder RNA von einem ersten Teil der Liganden (2, 6, 7) zu Basen der einzelsträngigen DNA (9) oder RNA von einem zweiten Teil der Liganden (3, 4, 5) komplementär sind und während dem Verfahren an die komplementären Basen binden, wobei eine Y-Form ausgebildet wird, und wobei während dem Verfahren eine Ligase zugegeben wird, welche die weitere einzelsträngige DNA oder RNA von dem ersten Teil der Liganden (2, 6, 7) mit der weiteren einzelsträngigen DNA (11) oder RNA von dem zweiten Teil der Liganden (3, 4, 5) chemisch kovalent ligiert.

9. Verfahren gemäß Anspruch 8, **dadurch gekennzeichnet, dass** bei einer einzelsträngigen DNA (8) oder RNA von einem ersten Teil der Liganden (2, 6, 7),
i) die den Ligand (2, 6, 7) an ihrem 5'-Ende aufweist, die Basen, die zu den Basen der einzelsträngigen DNA (9) oder RNA von einem zweiten Teil der Liganden (3, 4, 5) komplementär sind, an ihrem 5'-Ende aufweist und die 2 bis 10 Basen der weiteren einzelsträngigen DNA (10) oder RNA, die zu Basen der einzelsträngigen DNA (9) oder RNA von einem zweiten Teil der Liganden (3, 4, 5) komplementär sind, am 3'-Ende der weiteren einzelsträngigen DNA (10) oder RNA angeordnet sind; oder
ii) die den Ligand (2, 6, 7) an ihrem 3'-Ende aufweist, die Basen, die zu den Basen der einzelsträngigen DNA (9) oder RNA von einem zweiten Teil der Liganden (3, 4, 5) komplementär sind, an ihrem 3'-Ende aufweist und die 2 bis 10 Basen der weiteren einzelsträngigen DNA (10) oder RNA, die zu Basen der einzelsträngigen DNA (9) oder RNA von einem zweiten Teil der Liganden (3, 4, 5) komplementär sind, am 5'-Ende der weiteren einzelsträngigen DNA (10) oder RNA angeordnet sind.

10. Chemische Bibliothek, enthaltend Liganden (2, 3, 4, 5, 6, 7), die chemisch kovalent eine einzelsträngige DNA (8, 9) oder RNA mit einer Basenlänge von mehr als 2 Basen gebunden haben, wobei nur 2 bis 10 Basen der einzelsträngigen DNA (8) oder RNA von einem ersten Teil der Liganden (2, 6, 7) zu Basen der einzelsträngigen DNA (9) oder RNA von einem zweiten Teil der Liganden (3, 4, 5) komplementär sind, **dadurch gekennzeichnet, dass** die chemische Bibliothek binäre Komplexe der jeweiligen Liganden (2, 3, 4, 5, 6, 7) enthält, wobei die einzelsträngige DNA (8) oder RNA bei dem ersten Teil der Liganden (2, 6, 7) und die einzelsträngige DNA (9) oder RNA bei dem zweiten Teil der Liganden (3, 4, 5) eine Basensequenz enthält, die für den chemisch kovalent gebundenen Liganden (2, 3, 4, 5, 6, 7) kodiert.

11. Bibliothek gemäß Anspruch 10, **dadurch gekennzeichnet, dass** nur 3 bis 9 Basen, bevorzugt nur 5 bis 9 Basen, besonders bevorzugt nur 6 bis 8 Basen, der einzelsträngigen DNA (8) oder RNA von einem ersten Teil der Liganden (2, 6, 7) zu der einzelsträngigen DNA (9) oder RNA von einem zweiten Teil der Liganden (3, 4, 5) komplementär sind.

12. Bibliothek gemäß einem der Ansprüche 10 oder 11, **dadurch gekennzeichnet, dass** die Liganden (2, 3, 4, 5, 6, 7) ein Molekül ausgewählt aus der Gruppe bestehend aus Protein, Peptid, Lipid, Kohlenhydrat, dsDNA, ssDNA, dsRNA und ssRNA, Aptamer, organisches Molekül mit einer molekularen Masse ≤ 200 kDa, bevorzugt ≤ 100 kDa, besonders bevorzugt ≤ 10 kDa, insbesondere ≤ 3 kDa, enthalten oder daraus bestehen.

13. Bibliothek gemäß einem der Ansprüche 10 bis 12, **dadurch gekennzeichnet, dass** die einzelsträngige DNA (8, 9) oder RNA der Liganden der Bibliothek jeweils bereichsweise zu einer komplementären Basensequenz einer weiteren einzelsträngigen DNA (10, 11) oder RNA hybridisiert ist, wobei 2 bis 10 Basen einer ersten weiteren einzelsträngigen DNA (10) oder RNA von einem ersten Teil der Liganden (2, 6, 7) zu Basen der zweiten einzelsträngigen DNA (9) oder RNA von einem zweiten Teil der Liganden (3, 4, 5) komplementär sind.

14. Bibliothek gemäß Anspruch 13, **dadurch gekennzeichnet, dass** die einzelsträngigen DNA (8) oder RNA von einem ersten Teil der Liganden (2, 6, 7),
i) die den Ligand (2, 6, 7) an ihrem 5'-Ende aufweist, die Basen, die zu den Basen der einzelsträngigen DNA (9) oder RNA von einem zweiten Teil der Liganden (3, 4, 5) komplementär sind, an ihrem 5'-Ende aufweist und die 2 bis 10 Basen der weiteren einzelsträngigen DNA (10) oder RNA, die zu Basen der zweiten einzelsträngigen DNA (9) oder RNA von einem zweiten Teil der Liganden (3, 4, 5) komplementär sind, am 3'-Ende der weiteren einzelsträngigen DNA (10) oder RNA angeordnet sind; oder
ii) die den Ligand (2, 6, 7) an ihrem 3'-Ende aufweist, die Basen, die zu den Basen der einzelsträngigen DNA (9) oder RNA von einem zweiten Teil der Liganden (3, 4, 5) komplementär sind, an ihrem 3'-Ende aufweist und die 2 bis 10 Basen der weiteren einzelsträngigen DNA (10) oder RNA, die zu Basen der einzelsträngigen DNA (9) oder RNA von einem zweiten Teil der Liganden (3, 4, 5) komplementär sind, am 5'-Ende der weiteren einzelsträngigen DNA (10) oder RNA angeordnet sind.

15. Verwendung der Bibliothek gemäß einem der Ansprüche 10 bis 14 zur selektiven und sensitiven Identifizierung von hochaffinen ternären Liganden-Rezeptor-Komplexen.

## Claims

1. A method for sensitive identification of high-affinity complexes made of two ligands (2, 3, 4, 5, 6, 7) and one receptor (1), comprising the steps
i) interaction of a large number of different complexes made of ligands (2, 3, 4, 5, 6, 7) of a chemical library with at least one receptor (1) in a solution, the ligands (2, 3, 4, 5, 6, 7) of the library having a single-strand DNA (8, 9) or RNA with a base length of more than 2 bases, which is bonded chemically covalently to the ligands (2, 3, 4, 5, 6, 7), and only 2 to 10 bases of the single-strand DNA (8) or RNA of a first part of the ligands (2, 6, 7) being complementary to bases of the single-strand DNA (9) or RNA of a second part of the ligands (3, 4, 5);
ii) incubation of the solution for a specific period of time, complexes made of ligand complexes and the receptor (1) being produced;
iii) identification of the resulting complexes made of ligand complexes and the receptor (1),
**characterized in that** the solution is incubated at a room temperature at which, between the first part of the ligands (2, 6, 7) and the second part of the ligands (3, 4, 5), an equilibrium of hybridisation to form ligand complexes and dissociation to form free ligands (2, 3, 4, 5, 6, 7) arises, wherein, in step i), the ligands are complexed to form ligand complexes via hybridisation of the DNA (8, 9) or RNA, wherein the single-strand DNA (8) or RNA in the first part of the ligands (2, 6, 7) and the single-strand DNA (9) or RNA in the second part of the ligands (3, 4, 5) comprises a base sequence which codes for the chemically covalently-bonded ligand (2, 3, 4, 5, 6, 7).

2. The method according to the preceding claim, **characterized in that** only 3 to 9 bases, preferably only 5 to 9 bases, particularly preferably only 6 to 8 bases, of the single-strand DNA (8) or RNA of a first part of the ligands (2, 6, 7) are complementary to the single-strand DNA (9) or RNA of a second part of the ligands (3, 4, 5).

3. The method according to one of the preceding claims, **characterized in that**, in step ii), the solution is
a) incubated at a temperature of 1°C to 50°C, preferably 5 °C to 37 °C, particularly preferably 10 °C to 25 °C, especially 15 °C to 20 °C; and/or
b) incubated for a period of time of 0.1 to 48 hours, preferably 0.2 to 24 hours, particularly preferably 0.5 to 12 hours, especially 1 to 6 hours.

4. The method according to one of the preceding claims, **characterized in that** the receptor
i) is immobilized, preferably on a substrate selected from the group consisting of glass, ceramic, biopolymer, sepharose, synthetic polymer and hydrogel; and/or
ii) comprises or consists of a protein, a DNA, an RNA, a cell and/or an organic molecule with a molecular mass ≤ 200 kDa, preferably ≤ 100 kDa, particularly preferably ≤ 10 kDa, especially ≤ 3 kDa.

5. The method according to one of the preceding claims, **characterized in that** the ligands (2, 3, 4, 5, 6, 7) comprise or consist of a molecule selected from the group consisting of protein, peptide, lipid, carbohydrate, dsDNA, ssDNA, dsRNA and ssRNA, aptamer, organic molecule with a molecular mass ≤ 200 kDa, preferably ≤ 100 kDa, particularly preferably ≤ 10 kDa, especially ≤ 3 kDa.

6. The method according to one of the preceding claims, **characterized in that** the complexes made of ligand complexes and the receptor (1) are identified via an analytical method selected from the group consisting of mass spectrometry, HPLC, gas chromatography, IR spectroscopy and DNA sequencing, in particular via DNA sequencing of a DNA or RNA barcode.

7. The method according to one of the preceding claims, **characterized in that** the base sequence which codes for the chemically covalently bound ligand is hybridised at least partially to a complementary base sequence of a further single-stranded DNA or RNA.

8. The method according to one of the preceding claims, **characterized in that** the single-strand DNA (8, 9) or RNA of the ligands (2, 3, 4, 5, 6, 7) of the library is hybridised in regions to form a complementary base sequence of a further single-strand DNA (10, 11) or RNA, 2 to 10 bases of the further single-strand DNA (10) or RNA of a first part of the ligands (2, 6, 7) being complementary to bases of the single-strand DNA (9) or RNA of a second part of the ligands (3, 4, 5) and binding to the complementary bases during the method, a Y-form being formed, and a ligase being added during the method, which ligates chemically covalently the further single-strand DNA or RNA of the first part of the ligands (2. 6. 7) to the further single-strand DNA (11) or RNA of the second part of the ligands (3, 4, 5).

9. The method according to claim 8, **characterized in that**, in the case of a single-strand DNA (8) or RNA of a first part of the ligands (2, 6, 7),
i) which has the ligand (2, 6, 7) at the 5' end thereof, has the bases which are complementary to the bases of the single-strand DNA (9) or RNA of a second part of the ligands (3, 4, 5), at the 5' end thereof, and the 2 to 10 bases of the further single-strand DNA (10) or RNA, which are complementary to bases of the single-strand DNA (9) or RNA of a second part of the ligands (3, 4, 5), are disposed at the 3' end of the further single-strand DNA (10) or RNA; or
ii) which has the ligand (2, 6, 7) at the 3' end thereof, has the bases, which are complementary to the bases of the single-strand DNA (9) or RNA of a second part of the ligands (3, 4, 5), at the 3' end thereof, and the 2 to 10 bases of the further single-strand DNA (10) or RNA, which are complementary to bases of the single-strand DNA (9) or RNA of a second part of the ligands (3, 4, 5), are disposed at the 5' end of the further single-strand DNA (10) or RNA.

10. A chemical library, comprising ligands (2, 3, 4, 5, 6, 7) which have bonded chemically covalently a single-strand DNA (8, 9) or RNA with a base length of more than 2 bases, wherein only 2 to 10 bases of the single-strand DNA (8) or RNA of a first part of the ligands (2, 6, 7) are complementary to bases of the single-strand DNA (9) or RNA of a second part of the ligands (3, 4, 5), **characterized in that** the chemical library comprises binary complexes of the respective ligands (2, 3, 4, 5, 6, 7), the single-stranded DNA (8) or RNA in the first part of the ligands (2, 6, 7) and the single-stranded DNA (9) or RNA in the second part of the ligands (3, 4, 5) comprising a base sequence which codes for the chemically covalently bound ligand (2, 3, 4, 5, 6, 7)..

11. The library according to claim 10, **characterized in that** only 3 to 9 bases, preferably only 5 to 9 bases, particularly preferably only 6 to 8 bases, of the single-strand DNA (8) or RNA of a first part of the ligands (2, 6, 7) are complementary to the single-strand DNA (9) or RNA of a second part of the ligands (3, 4, 5).

12. The library according any one of claims 10 or 11, **characterized in that** the ligands (2, 3, 4, 5, 6, 7) comprise or consist of a molecule selected from the group consisting of protein, peptide, lipid, carbohydrate, dsDNA, ssDNA, dsRNA and ssRNA, aptamer, organic molecule with a molecular mass ≤ 200 kDa, preferably ≤ 100 kDa, particularly preferably ≤ 10 kDa, especially ≤ 3 kDa.

13. The library according to any one of claims 10 to 12, **characterized in that** the single-strand DNA (8, 9) or RNA of the ligands of the library is hybridised respectively in regions to a complementary base sequence of a further single-strand DNA (10, 11) or RNA, 2 to 10 bases of a first further single-strand DNA (10) or RNA of a first part of the ligands (2, 6, 7) being complementary to bases of the second single-strand DNA (9) or RNA of a second part of the ligands (3, 4, 5).

14. The library according to claim 13, **characterized in that** the single-strand DNA (8) or RNA of a first part of the ligands (2, 6, 7),
i) which has the ligand (2, 6, 7) at the 5' end thereof, has the bases, which are complementary to the bases of the single-strand DNA (9) or RNA of a second part of the ligands (3, 4, 5), at the 5' end thereof, and the 2 to 10 bases of the further single-strand DNA (10) or RNA, which are complementary to bases of the second single-strand DNA (9) or RNA of a second part of the ligands (3, 4, 5), are disposed at the 3' end of the further single-strand DNA (10) or RNA; or
ii) which has the ligand (2, 6, 7) at the 3' end thereof, has the bases, which are complementary to the bases of the single-strand DNA (9) or RNA of a second part of the ligands (3, 4, 5), at the 3' end thereof, and the 2 to 10 bases of the further single-strand DNA (10) or RNA, which are complementary to bases of the single-strand DNA (9) or RNA of a second part of the ligands (3, 4, 5), are disposed at the 5' end of the further single-strand DNA (10) or RNA.

15. A use of the library according to any one of claims 10 to 14 for selective and sensitive identification of high-affinity ternary ligand-receptor complexes.

## Revendications

1. Procédé d'identification sensible de complexes à haute affinité composés de deux ligands (2, 3, 4, 5, 6, 7) et d'un récepteur (1), comprenant les étapes ci-dessous consistant à :
i) faire interagir un grand nombre de complexes différents issus de ligands (2, 3, 4, 5, 6, 7) d'une bibliothèque chimique avec au moins un récepteur (1) dans une solution, dans lequel les ligands (2, 3, 4, 5, 6, 7) de la bibliothèque présentent un ADN (8, 9), ou ARN, monocaténaire présentant une longueur de base de plus de 2 bases, qui est lié de manière chimiquement covalente aux ligands (2, 3, 4, 5, 6, 7) et dans lequel seulement 2 à 10 bases de l'ADN (8), ou ARN, monocaténaire d'une première partie des ligands (2, 6, 7) sont complémentaires aux bases de l'ADN (9), ou ARN, monocaténaire d'une deuxième partie des ligands (3, 4, 5) ;
ii) faire incuber la solution pendant une période déterminée, ce qui fait apparaitre des complexes issus des complexes de ligands et du récepteur (1) ;
iii) identifier les complexes apparus issus des complexes de ligands et du récepteur (1),
**caractérisé en ce que** la solution est incubée à une température ambiante dans laquelle un équilibre est établi entre la première partie des ligands (2, 6, 7) et la deuxième partie des ligands (3, 4, 5) grâce à l'hybridation en complexes de ligands et à la dissociation en ligands libres (2, 3, 4, 5, 6, 7), dans lequel, à l'étape i), les ligands sont complexés en complexes de ligands par hybridation de l'ADN (8, 9) ou de l'ARN, dans lequel l'ADN (8), ou ARN, monocaténaire, pour ce qui est de la première partie des ligands (2, 6, 7), et l'ADN (9), ou ARN, monocaténaire, pour ce qui est de la deuxième partie des ligands (3, 4, 5), contient une séquence de base qui code pour les ligands (2, 3, 4, 5, 6, 7) liés de manière chimiquement covalente.

2. Procédé selon la revendication précédente, **caractérisé en ce que** seulement 3 à 9 bases, de manière préférée seulement 5 à 9 bases, de manière particulièrement préférée seulement 6 à 8 bases, de l'ADN (8), ou ARN, monocaténaire d'une première partie des ligands (2, 6, 7) sont complémentaires à l'ADN (9), ou ARN, monocaténaire d'une deuxième partie des ligands (3, 4, 5).

3. Procédé selon l'une quelconque des revendications précédentes, **caractérisé en ce que** la solution, à l'étape ii),
a) est incubée à une température comprise entre 1°C et 50°C, de manière préférée comprise entre 5°C et 37°C, de manière particulièrement préférée comprise entre 10°C et 25°C, en particulier comprise entre 15 et 20°C ; et/ou
b) est incubée pendant une période comprise entre 0,1 et 48 heures, de manière préférée comprise entre 0,2 et 24 heures, de manière particulièrement préférée comprise entre 0,5 et 12 heures, en particulier comprise entre 1 et 6 heures.

4. Procédé selon l'une quelconque des revendications précédentes, **caractérisé en ce que** le récepteur (1)
i) est immobilisé, de manière préférée sur un substrat choisi dans le groupe constitué d'un verre, d'une céramique, d'un biopolymère, de sépharose, d'un polymère synthétique et d'un hydrogel ; et/ou
ii) contient ou est constitué d'une protéine, d'un ADN, d'un ARN, d'une cellule et/ou d'une molécule organique présentant une masse moléculaire ≤ 200 kDa, de manière préférée ≤ 100 kDa, de manière particulièrement préférée ≤ 10 kDa, en particulier ≤ 3 kDa.

5. Procédé selon l'une quelconque des revendications précédentes, **caractérisé en ce que** les ligands (2, 3, 4, 5, 6, 7) contiennent ou sont constitués d'une molécule choisie dans le groupe constitué d'une protéine, d'un peptide, d'un lipide, d'un hydrate de carbone, d'un ADNdb, d'un ADNsb, d'un ARNdb et d'un ARNsb, d'un aptamère, d'une molécule organique présentant une masse moléculaire ≤ 200 kDa, de manière préférée ≤ 100 kDa, de manière particulièrement préférée ≤ 10 kDa, en particulier ≤ 3 kDa.

6. Procédé selon l'une quelconque des revendications précédentes, **caractérisé en ce que** les complexes issus de complexes de ligands et du récepteur (1) sont identifiés par un procédé analytique, de manière préférée choisi dans le groupe constitué de la spectrométrie de masse, de la CLHP, de la chromatographie en phase gazeuse, de la spectroscopie IR et du séquençage de l'ADN, en particulier par séquençage ADN d'un code-barres ADN ou ARN.

7. Procédé selon l'une quelconque des revendications précédentes, **caractérisé en ce que** la séquence de bases qui code pour le ligand lié de manière chimiquement covalente est au moins localement hybridée en une séquence de bases complémentaire à un autre ADN ou ARN monocaténaire.

8. Procédé selon l'une quelconque des revendications précédentes, **caractérisé en ce que** l'ADN (8, 9), ou ARN, monocaténaire des ligands (2, 3, 4, 5, 6, 7) de la bibliothèque est hybridé localement en une séquence de bases complémentaire à un autre ADN (10, 11), ou ARN, monocaténaire, dans lequel 2 à 10 bases de l'autre ADN (10), ou ARN, monocaténaire d'une première partie des ligands (2, 6, 7) sont complémentaires aux bases de l'ADN (9), ou ARN, monocaténaire d'une deuxième partie des ligands (3, 4, 5) et se lient aux bases complémentaires au cours du procédé, une forme en Y étant créée, et dans lequel une ligase est ajoutée au cours du procédé, laquelle suture de manière chimiquement covalente l'autre ADN, ou ARN, monocaténaire de la première partie des ligands (2, 6, 7) avec l'autre ADN (11), ou ARN, monocaténaire de la deuxième partie des ligands (3, 4, 5).

9. Procédé selon la revendication 8, **caractérisé en ce que** l'ADN (8), ou ARN, monocaténaire d'une première partie des ligands (2, 6, 7),
i) qui présente le ligand (2, 6, 7) à son extrémité 5', présente à son extrémité 5' les bases qui sont complémentaires aux bases de l'ADN (9), ou ARN, monocaténaire d'une deuxième partie des ligands (3, 4, 5), et les 2 à 10 bases de l'autre ADN (10), ou ARN, monocaténaire qui sont complémentaires aux bases de l'ADN (9), ou ARN, monocaténaire d'une deuxième partie des ligands (3, 4, 5) sont agencées à l'extrémité 3' de l'autre ADN (10), ou ARN, monocaténaire ; ou
ii) qui présente le ligand (2, 6, 7) à son extrémité 3', présente à son extrémité 3' les bases qui sont complémentaires aux bases de l'ADN (9), ou ARN, monocaténaire d'une deuxième partie des ligands (3, 4, 5), et les 2 à 10 bases de l'autre ADN (10), ou ARN, monocaténaire qui sont complémentaires aux bases de l'ADN (9), ou ARN, monocaténaire d'une deuxième partie des ligands (3, 4, 5) sont agencées à l'extrémité 5' de l'autre ADN (10), ou ARN, monocaténaire.

10. Bibliothèque chimique, contenant des ligands (2, 3, 4, 5, 6, 7) qui ont lié de manière chimiquement covalente un ADN (8, 9), ou ARN, monocaténaire présentant une longueur de base de plus de 2 bases, dans laquelle seulement 2 à 10 bases de l'ADN (8), ou ARN, monocaténaire d'une première partie des ligands (2, 6, 7) sont complémentaires aux bases de l'ADN (9), ou ARN, monocaténaire d'une deuxième partie des ligands (3, 4, 5), **caractérisée en ce que** la bibliothèque chimique contient des complexes binaires des ligands (2, 3, 4, 5, 6, 7) respectifs, dans laquelle l'ADN (8), ou ARN, monocaténaire, pour ce qui est de la première partie des ligands (2, 6, 7), et l'ADN (9), ou ARN, monocaténaire, pour ce qui est de la deuxième partie des ligands (3, 4, 5) contient une séquence de bases qui code pour les ligand (2, 3, 4, 5, 6, 7) liés de manière chimiquement covalente.

11. Bibliothèque selon la revendication précédente, **caractérisé en ce que** seulement 3 à 9 bases, de manière préférée seulement 5 à 9 bases, de manière particulièrement préférée seulement 6 à 8 bases, de l'ADN (8), ou ARN, monocaténaire d'une première partie des ligands (2, 6, 7) sont complémentaires à l'ADN (9), ou ARN, monocaténaire d'une deuxième partie des ligands (3, 4, 5).

12. Bibliothèque selon la revendication 10 ou 11, **caractérisée en ce que** les ligands (2, 3, 4, 5, 6, 7) contiennent ou sont constitués d'une molécule choisie dans le groupe constitué d'une protéine, d'un peptide, d'un lipide, d'un glucide, d'un ADNdb, d'un ADNsb, d'un ARNdb et d'un ARNsb, d'un aptamère, d'une molécule organique présentant une masse moléculaire ≤ 200 kDa, de manière préférée ≤ 100 kDa, de manière particulièrement préférée ≤ 10 kDa, en particulier ≤ 3 kDa.

13. Bibliothèque selon l'une quelconque des revendications 10 à 12, **caractérisée en ce que** l'ADN (8, 9), ou ARN, monocaténaire des ligands de la bibliothèque est respectivement hybridé localement en une séquence de bases complémentaire à un autre ADN (10, 11), ou ARN, monocaténaire, dans lequel 2 à 10 bases d'un premier autre ADN (10), ou ARN, monocaténaire d'une première partie des ligands (2, 6, 7) sont complémentaires aux bases du deuxième ADN (9), ou ARN, monocaténaires d'une deuxième partie des ligands (3, 4, 5).

14. Bibliothèque selon la revendication 13, **caractérisée en ce que** l'ADN (8), ou ARN, monocaténaire d'une première partie des ligands (2, 6, 7),
i) qui présente le ligand (2, 6, 7) à son extrémité 5', présente à son extrémité 5' les bases qui sont complémentaires aux bases de l'ADN (9), ou ARN, monocaténaire d'une deuxième partie des ligands (3, 4, 5), et les 2 à 10 bases de l'autre ADN (10), ou ARN, monocaténaire qui sont complémentaires aux bases de l'ADN (9), ou ARN, monocaténaire d'une deuxième partie des ligands (3, 4, 5) sont agencées à l'extrémité 3' de l'autre ADN (10), ou ARN, monocaténaire ; ou
ii) qui présente le ligand (2, 6, 7) à son extrémité 3', présente à son extrémité 3' les bases qui sont complémentaires aux bases de l'ADN (9), ou ARN, monocaténaire d'une deuxième partie des ligands (3, 4, 5), et les 2 à 10 bases de l'autre ADN (10), ou ARN, monocaténaire qui sont complémentaires aux bases de l'ADN (9), ou ARN, monocaténaire d'une deuxième partie des ligands (3, 4, 5) sont agencées à l'extrémité 5' de l'autre ADN (10), ou ARN, monocaténaire.

15. Utilisation de la bibliothèque selon l'une quelconque des revendications 10 à 14 pour l'identification sélective et sensible de complexes ligands-récepteur ternaires à haute affinité.
